(19) **Europäisches Patentamt / European Patent Office / Office européen des brevets**

(11) **EP 3 269 308 B1**

(12) **EUROPEAN PATENT SPECIFICATION**

(45) Date of publication and mention
of the grant of the patent:
**23.10.2019 Bulletin 2019/43**

(21) Application number: **16761389.2**

(22) Date of filing: **03.02.2016**

(51) Int Cl.:
**A61B 8/08** (2006.01)    **A61B 8/00** (2006.01)

(86) International application number:
**PCT/JP2016/053150**

(87) International publication number:
**WO 2016/143417 (15.09.2016 Gazette 2016/37)**

(54) **ULTRASONIC DIAGNOSTIC DEVICE**

ULTRASCHALLDIAGNOSEVORRICHTUNG

DISPOSITIF ULTRASONORE DE DIAGNOSTIC

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.03.2015 JP 2015048230**

(43) Date of publication of application:
**17.01.2018 Bulletin 2018/03**

(73) Proprietor: **Hitachi, Ltd.
Tokyo 100-8280 (JP)**

(72) Inventors:
• **SONOYAMA, Teruyuki
Tokyo 181-8622 (JP)**

• **INOUE, Noriaki
Tokyo 181-8622 (JP)**

(74) Representative: **Strehl Schübel-Hopf & Partner
Maximilianstrasse 54
80538 München (DE)**

(56) References cited:
WO-A1-2011/004661    WO-A1-2015/029651
WO-A1-2016/060146    JP-A- 2014 000 260
JP-A- 2014 042 637    US-A1- 2010 016 718
US-A1- 2013 218 011    US-A1- 2015 005 633
US-B2- 8 118 744

## Description

TECHNICAL FIELD

The present disclosure relates to an ultrasound diagnostic apparatus, and in particular to a technique for measuring a shear wave.

BACKGROUND

There are known ultrasound diagnostic apparatuses which measure a displacement of a tissue in a subject and obtain diagnosis information from within the subject. For example, ultrasound may be transmitted to generate a shear wave within a subject, a displacement of a tissue due to propagation of the shear wave may be measured with ultrasound, and diagnosis information such as hardness of the tissue within the subject may be obtained based on a measurement value such as a propagation velocity of the shear wave.

For example, Patent Document 1 discloses an invention in which displacements of a shear wave are measured at a plurality of positions different from each other, and a propagation velocity of the shear wave is calculated based on time at each position when a maximum displacement is obtained.

CITATION LIST

PATENT LITERATURE

Patent Document 1: US Patent No. 8,118,744
US 2013/218011 A1 describes an ultrasonic device with the features in the preamble of present claim 1. Another conventional device is disclosed in US 2015/005633 A1. A further ultrasonic device is shown in WO 2016/060146 A1 which has been published after the filing date of the present application.

SUMMARY

TECHNICAL PROBLEM

It is difficult to judge whether or not a measurement value is reliable by simply displaying the measurement value of the shear wave (such as the propagation velocity) in the tissue obtained using ultrasound. For example, when the propagation velocity of the shear wave is measured for each depth of a plurality of depths in the subject; that is, when a plurality of propagation velocities corresponding to the plurality of depths are obtained, if there is a variation among the plurality of propagation velocities, it is difficult to judge whether the variation is a reliable variation reflecting tissue characteristics for each depth, or an unreliable variation due to instability of the measurement state or the like.

Because of this, it is desired to not only simply display the measurement value of the shear wave (such as the propagation velocity), but also be able to evaluate, for example, the reliability of the measurement value or the like.

The present invention was conceived in view of the above-described background and circumstances, and an advantage thereof lies in provision of an improved technique for evaluating the measurement value of the shear wave measured using ultrasound.

SOLUTION TO PROBLEM

The invention is defined in appended claim 1. The dependent claims relate to preferred embodiments.
According to one aspect of the present disclosure, there is provided an ultrasound diagnostic apparatus comprising: an ultrasound probe that transmits an ultrasound push wave to generate a shear wave in a subject, and that transmits an ultrasound tracking wave to the subject; a shear wave measurement unit that obtains a measurement value array including a measurement value at each depth of a plurality of depths within the subject by measuring the shear wave based on a reception signal obtained by transmitting the tracking wave; and a measurement value processor that identifies a measurement value which satisfies a discard condition from among a plurality of the measurement value arrays obtained by executing the measurement of the shear wave a plurality of times.

The discard condition in the above-described apparatus is desirably a condition based on a size of the measurement value, a condition based on a tissue state in the subject, or the like, for example. By a condition based on the size of the measurement value, for example, measurement values which are not appropriate are set as a discarding target. Similarly, by a condition based on the tissue state in the subject, for example, measurement values at a region (such as the depth) where superior measurement cannot be executed are set as discarding targets.

According to the above-described apparatus, the measurement value of the shear wave can be evaluated based on the discard conditions. For example, it becomes possible to identify reliable measurement values which do not satisfy the discard condition (or which do not correspond to the discard condition), by assuming that the measurement values which satisfy the discard condition (or which correspond to the discard condition) are not reliable.

According to another aspect of the present disclosure, the ultrasound diagnostic apparatus further comprises a detector that detects a fluctuation which is a periodic displacement of a tissue in the subject, based on the reception signal, wherein the discard condition includes a condition to discard a measurement value of each depth in which the fluctuation is detected, and the measurement value processor discards the measurement value of each depth in which the fluctuation is detected, from among the plurality of measurement values of the plurality of measurement value arrays.

According to another aspect of the present disclosure, the discard condition includes a condition to discard a measurement value which is outside of a threshold range, and the measurement value processor discards the measurement values which are outside of the threshold range, from among the plurality of measurement values of the plurality of measurement value arrays.

According to another aspect of the present disclosure, the measurement value processor discards a measurement value which satisfies the discard condition, from among the plurality of measurement values of the plurality of measurement value arrays, and calculates a ratio of a plurality of measurement values which are not discarded and which remain.

According to another aspect of the present disclosure, the measurement value processor discards a measurement value which satisfies the discard condition, from among the plurality of measurement values of the plurality of the measurement value arrays, and calculates a statistical value based on a plurality of measurement values which are not discarded and which remain.

According to another aspect of the present disclosure, the measurement value processor discards a measurement value which satisfies the discard condition, from among the plurality of measurement values of the plurality of measurement value arrays, and forms a histogram of a plurality of measurement values which are not discarded and which remain.

ADVANTAGEOUS EFFECTS OF INVENTION

According to various aspects of the present disclosure, there can be provided an improved technique for evaluating the measurement value of the shear wave measured using ultrasound. For example, according to an embodiment of the present disclosure, the measurement value of shear wave can be evaluated based on a discard condition.

BRIEF DESCRIPTION OF DRAWINGS

FIG. 1 is a diagram showing an overall structure of an ultrasound diagnostic apparatus desirable in an embodiment of the present disclosure.
FIG. 2 is a diagram for explaining a specific example related to generation of a shear wave and measurement of a displacement.
FIG. 3 is a diagram showing a specific example of a time-space map.
FIG. 4 is a diagram showing a specific example of a fluctuation.
FIG. 5 is a diagram for explaining a specific example of detection of fluctuation.
FIG. 6 is a diagram showing a specific example of a measurement result of a measurement set.

FIG. 7 is a diagram for explaining a specific example of a discard condition.
FIG. 8 is a diagram showing a specific example of a histogram related to a propagation velocity Vs.
FIG. 9 is a diagram showing a specific example of a display image.

DESCRIPTION OF EMBODIMENTS

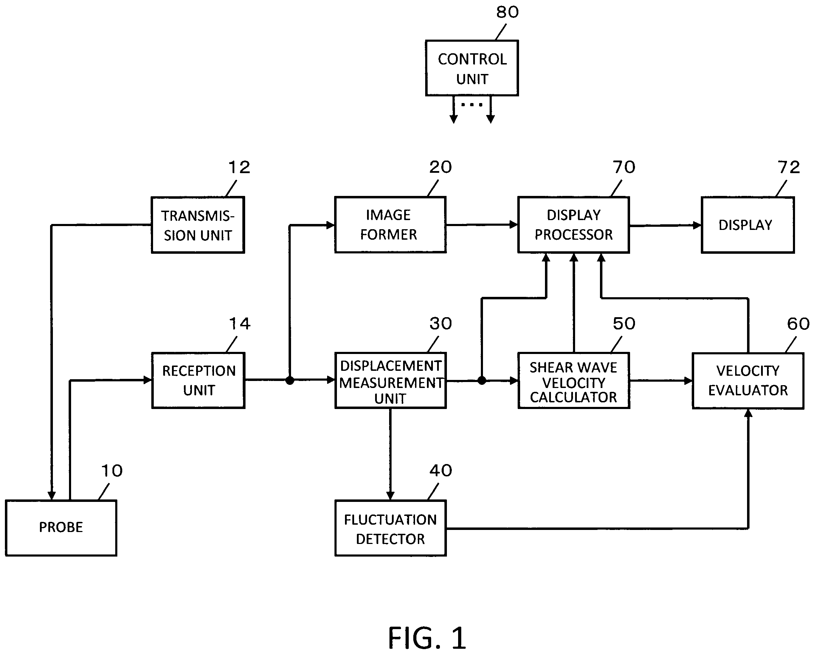

FIG. 1 is a diagram showing an overall structure of an ultrasound diagnostic apparatus desirable in an embodiment of the present disclosure. A probe 10 is an ultrasound probe which transmits and receives ultrasound to and from a region including diagnosis target such as a tissue within a subject (living body) such as, for example, an organ. The probe 10 comprises a plurality of transducer elements, each of which transmits and receives ultrasound, or transmits the ultrasound, and the plurality of transducer elements are transmission-controlled by a transmission unit 12, to form a transmission beam.

The plurality of transducer elements of the probe 10 receive ultrasound from within a region including the diagnosis target, and a signal obtained by the reception is output to a reception unit 14. The reception unit 14 forms a reception beam, and a reception signal (echo data) is collected along the reception beam. The probe 10 is desirably, for example, of a convex type, but may alternatively be of a linear type or the like.

The probe 10 has a function to transmit ultrasound which generates a shear wave in the region including the diagnosis target tissue (push wave), a function to transmit and receive ultrasound for measuring a displacement of the tissue due to the shear wave (tracking wave), and a function to transmit and receive ultrasound for image formation.

The transmission of the ultrasound is controlled by the transmission unit 12. When the shear wave is to be generated, the transmission unit 12 outputs a transmission signal of the push wave to the plurality of transducer elements of the probe 10, and a transmission beam of the push wave is consequently formed. When the shear wave is to be measured, the transmission unit 12 outputs a transmission signal of the tracking wave to the plurality of transducer elements of the probe 10, and a transmission beam of the tracking wave is consequently formed. When an ultrasound image is to be formed, the transmission unit 12 outputs a transmission signal for image formation to the plurality of transducer elements of the probe 10, and a transmission beam for image formation is consequently scanned.

The reception unit 14 forms a reception beam of the tracking wave based on a reception wave signal obtained from the plurality of transducer elements by the probe 10 transmitting and receiving the tracking wave, and obtains a reception signal corresponding to the reception beam. Further, the reception unit 14 forms a reception beam for image formation based on a reception wave signal obtained from the plurality of transducer elements by the probe 10 transmitting and receiving ultrasound for image formation, and generates a reception signal corresponding to the reception beam.

The ultrasound beams for image formation (the transmission beam and the reception beam) are scanned within a two-dimensional plane including the diagnosis target, and a reception signal for image formation is collected from within the two-dimensional plane. Alternatively, the ultrasound beam for image formation may be three-dimensionally scanned within a three-dimensional space, and a reception signal for image formation may be collected from within the three-dimensional space.

An image former 20 forms ultrasound image data based on the reception signal for image formation collected at the reception unit 14. For example, the image former 20 forms image data of a B-mode image (tomographic image) of a region including the diagnosis target tissue such as the organ. When the reception signal for image formation is collected three-dimensionally, the image former 20 may form image data of a three-dimensional ultrasound image.

A displacement measurement unit 30 measures a displacement of a tissue after generation of the shear wave in the subject based on the reception signal corresponding to the reception beam of the tracking wave obtained from the reception unit 14. A fluctuation detector 40 detects a periodic displacement based on a measurement result of the displacement obtained from the displacement measurement unit 30. A shear wave velocity calculator 50 calculates a propagation velocity of the shear wave within the subject based on the measurement result obtained from the displacement measurement unit 30. A velocity evaluator 60 evaluates the propagation velocity calculated by the shear wave velocity calculator 50. In the evaluation, a detection result obtained from the fluctuation detector 40 is also referred to. Processes at the displacement measurement unit 30, the fluctuation detector 40, the shear wave velocity calculator 50, and the velocity evaluator 60 will be described later in detail.

A display processor 70 forms a display image based on the image data of the ultrasound image obtained from the image former 20, the velocity information obtained from the shear wave velocity calculator 50, the measurement result obtained from the displacement measurement unit 30, and an evaluation result obtained from the velocity evaluator 60. The display image formed by the display processor 70 is displayed on a display 72.

A control unit 80 controls the entirety of the ultrasound diagnostic apparatus shown in FIG. 1. The ultrasound diagnostic apparatus of FIG. 1 desirably comprises an operation device including, for example, a mouse, a keyboard, a trackball, a touch panel, and other switches. In the overall control by the control unit 80, an instruction received from a user via the operation device or the like is also reflected.

Of the structures shown in FIG. 1 (function blocks assigned with reference numerals), the transmission unit 12, the reception unit 14, the image former 20, the displacement measurement unit 30, the fluctuation detector 40, the shear wave velocity calculator 50, the velocity evaluator 60, and the display processor 70 may be realized using hardware such as, for example, an electric/electronic circuit, a processor, or the like, and a device such as a memory may be used as necessary in realizing these units. Alternatively, the functions corresponding to these units may be realized by a cooperation of hardware such as a CPU, a processor, and a memory, and software (program) which defines operations of the CPU and the processor. A desirable specific example of the display 72 is a liquid crystal display or the like. The control unit 80 may be realized, for example, by cooperation of hardware such as a CPU, a processor, and a memory, and software (program) which defines operations of the CPU and the processor.

The overall structure of the ultrasound diagnostic apparatus of FIG. 1 has been described. Next, generation of a shear wave, measurement of a displacement, or the like by the ultrasound diagnostic apparatus of FIG. 1 will be described in detail. In the following description, for the structures (functional blocks) shown in FIG. 1, reference numerals of FIG. 1 will be referred to.

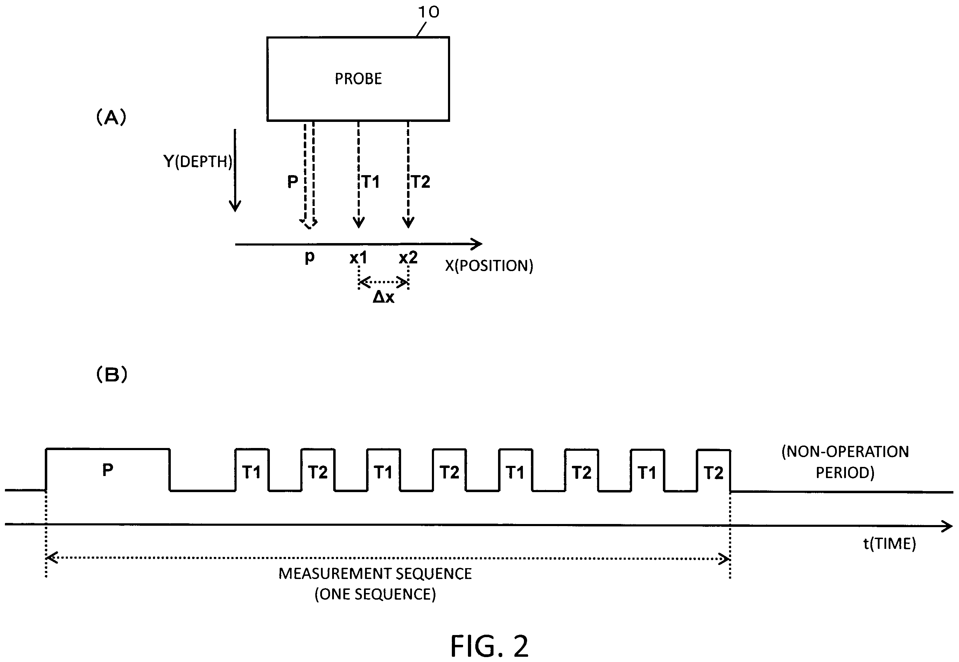

FIG. 2 is a diagram for explaining a specific example related to the generation of the shear wave and the measurement of the displacement. FIG. 2(A) shows a specific example of a transmission beam P of the push wave and ultrasound beams T1 and T2 for the tracking wave, formed using the probe 10.

In FIG. 2(A), the transmission beam P of the push wave is formed along a depth Y direction, to pass a position p in an X direction. For example, the transmission beam P of the push wave is formed with a position p on an X-axis shown in FIG. 2(A) as a focal point. The position p is set at a desired position, for example, by a user (inspector) such as a doctor or an inspection technician viewing the ultrasound image related to the diagnosis target in the living body displayed on the display 72.

When the transmission beam P is formed with the position p as the focal point, and the push wave is transmitted, a relatively strong shear wave is generated at the position p and a region nearby. FIG. 2(A) shows a specific example of measuring the propagation velocity in the X direction of the shear generated at the position p.

In the specific example of FIG. 2(A), two ultrasound beams T1 and T2 for the tracking wave are formed. The ultrasound beams (the transmission beam and the reception beam) T1 are formed, for example, to pass through a position x1 on the X-axis shown in FIG. 2(A), and the ultrasound beams (the transmission beam and the reception beam) T2 are formed, for example, to pass through a position x2 on the X-axis shown in FIG. 2(A). The position x1 and the position x2 may be set, for example, to desired positions by a user viewing the ultrasound image of the diagnosis target displayed on the display 72, or may be set by the ultrasound diagnosis apparatus of FIG. 1 at locations distanced by a predetermined distance along the X direction from the position p.

FIG. 2(B) shows a specific example of generation timings of the transmission beam P of the push wave and the ultrasound beams T1 and T2 of the tracking wave. A horizontal axis of FIG. 2(B) is a time axis t.

In FIG. 2(B), a period P is a period in which the transmission beam P of the push wave is formed, and periods T1 and T2 are respectively periods in which the ultrasound beams T1 and T2 for the tracking wave are formed.

During the period P, a large number of push waves are transmitted. For example, during the period P, ultrasound of a continuous wave is transmitted. Then, for example, from a time immediately after the completion of the period P, the shear wave is generated at the position p.

In the periods T1 and T2, tracking waves in a form of a pulse wave having one wave to several waves are transmitted, and a reflection wave of the pulse wave is received. For example, ultrasound beams T1 and T2 passing through the positions x1 and x2 are formed, and reception signals are obtained at a plurality of depths including the positions x1 and x2. In other words, for each of the ultrasound beams T1 and T2, reception signals are obtained from a plurality of depths.

The transmission and reception of the tracking wave are repeatedly executed for a plurality of periods. In other words, as shown in FIG. 2(B), the periods T1 and T2 are alternately repeated until, for example, a displacement of the tissue due to the shear wave is observed.

The displacement measurement unit 30 forms a time-space map related to the ultrasound beam T1 based on the reception signal of the ultrasound beam T1 of the tracking wave, and forms a time-space map related to the ultrasound beam T2 based on the reception signal of the ultrasound beam T2 of the tracking wave.

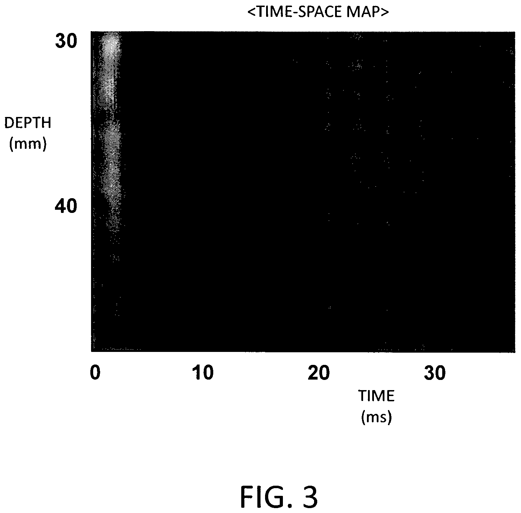

FIG. 3 is a diagram showing a specific example of the time-space map. The displacement measurement unit 30 calculates a phase shift of the reception signal at a plurality of depths (a plurality of locations in the depth direction) based on the reception signal of the ultrasound beam T1 of the tracking wave. The displacement measurement unit 30 calculates a phase shift of the reception signal (a derivative of the phase) over a plurality of times for each depth. The displacement measurement unit 30 forms the time-space map having a horizontal axis as a time (time axis) and a vertical axis as the depth, and mapping the phase shift of the reception signal.

In the specific example of the time-space map shown in FIG. 3, the phase shift of the reception signal is represented by brightness in the time-space map. For example, a higher brightness (white) is assigned to the phase shift in a positive direction and having a larger absolute value, and a lower brightness (black) is assigned to the phase shift in a negative direction and having a larger absolute value. In the specific example of FIG. 3, during a period of time 0 (zero) to 10 ms (milliseconds), the phase shift relatively largely changes from a high brightness (white) to a low brightness (black), indicating that a shear wave has passed during this period.

The time-space map of FIG. 3 is merely one specific example, and the phase shift of the reception signal may alternatively be represented by display forms other than the brightness; for example, by color. For example, a color based on red is assigned for a phase shift in the positive direction and having a larger absolute value, a color based on green is assigned for a phase shift closer to zero, and a color based on blue is assigned for a phase shift in the negative direction and having a larger absolute value.

In this manner, the displacement measurement unit 30 forms the time-space map related to the ultrasound beam T1 based on the reception signal of the ultrasound beam T1 of the tracking wave. Further, the displacement measurement unit 30 calculates a phase shift of the reception signal at a plurality of depths based on the reception signal of the ultrasound beam T2 of the tracking wave, and forms a time-space map related to the ultrasound beam T2.

Referring back to FIG. 2, the shear wave velocity calculator 50 calculates a propagation velocity Vs in the X-axis direction of the shear wave based on the phase shifts at the position x1 and the position x2 which change due to the influence of the shear wave generated at the position p. For example, the propagation velocity in the X-axis direction of the shear wave Vs is calculated as Vs = Δx/(t2 - t1) based on a time t1 when the phase shift is at the maximum at the position x1, a time t2 when the phase shift is at the maximum at the position x2, and a distance Δx between the position x1 and the position x2. Alternatively, the propagation velocity of the shear wave may be calculated by other known methods.

The shear wave velocity calculator 50 calculates the propagation velocity Vs for each depth of the plurality of depths based on, for example, the time-space maps (FIG. 3) of the ultrasound beam T1 and the ultrasound beam T2. Further, based on the propagation velocity Vs of the shear wave, elasticity information such as an elasticity value of a tissue in which the shear wave is measured may be calculated, or a viscoelastisity parameter, attenuation, a frequency characteristic, or the like may be derived as information of the tissue.

A measurement sequence shown in FIG. 2(B) is a period from start of transmission of the push wave until the calculation of the propagation velocity of the shear wave. After the completion of the measurement sequence, desirably, a non-operation period for allowing the probe 10 to cool is provided. In addition, after the non-operation period, a next measurement sequence may be further started.

In the specific example of FIG. 2, the ultrasound beams T1 and T2 of the tracking wave are formed in the positive direction side of the X-axis, for the transmission beam P of the push wave. Alternatively, the ultrasound beams T1 and T2 of the tracking wave may be formed in the negative direction side of the X-axis, for the transmission beam P of the push wave, and a shear wave propagating in the negative direction side of the X-axis may be measured. Desirably, the position p of the transmission beam P of the push wave and the positions x1 and x2 of the ultrasound beams T1 and T2 of the tracking wave are suitably set according to the diagnosis target, a diagnosis situation, or the like.

In the measurement of the propagation velocity of the shear wave, there may be cases where the displacement of the tissue is periodically fluctuated due to a motion of micro-blood vessels and bloodstream in a measurement region (region of interest), which may affect the measurement of the propagation velocity of the shear wave.

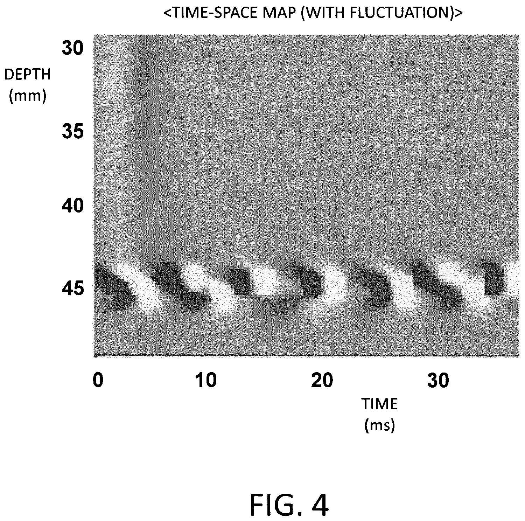

FIG. 4 is a diagram showing a specific example of the fluctuation. FIG. 4 shows a specific example of a time-space map which is obtained when a fluctuation occurs. Compared to the time-space map of FIG. 3, in the time-space map of FIG. 4, the fluctuation occurs near a depth of 45 mm (millimeters). Specifically, near the depth of 45 mm, the phase shift of the reception signal is periodically repeatedly changed between a low brightness (black) and a high brightness (white) over a relatively long period (0 ~ 30 ms or longer), and the phase shift is periodically fluctuated.

Because of this, near the depth of 45 mm, it is not possible to identify the change of the phase shift due to passage of the shear wave, and the propagation velocity of the shear wave cannot be measured. Even if the propagation velocity of the shear wave can be measured in the region (depth) where the fluctuation occurs, the reliability of the measurement result is questionable.

In consideration of this, the fluctuation detector 40 detects the fluctuation which is a periodical displacement, based on a measurement result of the displacement by the displacement measurement unit 30.

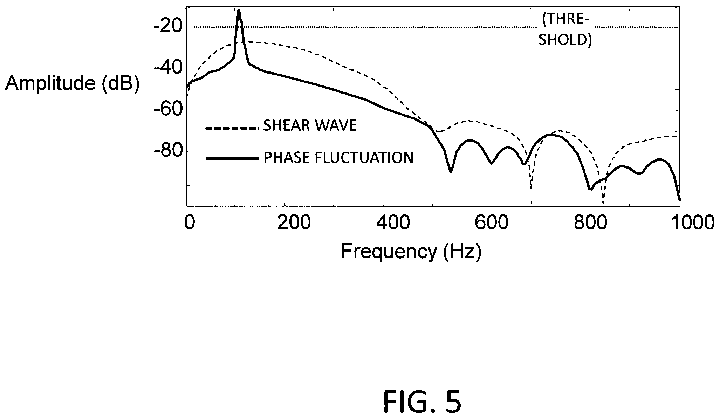

FIG. 5 is a diagram for explaining a specific example of detection of fluctuation. The fluctuation detector 40 frequency-analyzes a temporal change of the phase shift at each depth based on the time-space map obtained from the displacement measurement unit 30, and checks whether or not there is a frequency component which corresponds to fluctuation.

FIG. 5 shows a result of frequency analysis of the temporal change of the phase shift. In FIG. 5, a horizontal axis shows frequency (Hz; hertz), and a vertical axis represents the intensity of a power spectrum; that is, intensity of each frequency component (dB; decibels).

FIG. 5 shows a frequency spectrum (solid line) of a "phase fluctuation" at a depth where fluctuation occurs, and a frequency spectrum (broken line) of a "shear wave" at a depth where fluctuation does not occur.

In the frequency spectrum of the "phase fluctuation," a peak (maximum) having a significantly large intensity appears near a particular frequency; in the specific example of FIG. 5, near 100 Hz. On the other hand, in the frequency spectrum of the "shear wave" which does not include the fluctuation, no significantly large peak as in the "phase fluctuation" appears. The fluctuation detector 40 judges that the displacement at a certain depth is periodic and a fluctuation occurring at this depth when a peak with a significantly large intensity exists in the frequency spectrum of the phase change at each depth. The fluctuation detector 40 judges, for example, that fluctuation occurred at the depth when there is a peak having an intensity that exceeds a threshold within the frequency spectrum of the phase change at each depth.

Alternatively, the fluctuation detector 40 may detect the fluctuation by a process different from frequency analysis. For example, in the time-space map, absolute values of the phase shift may be added over a plurality of times for each depth, and the depth where the fluctuation occurs may be identified based on an addition result obtained for each depth. As exemplified in FIG. 4, at the depth where the fluctuation occurs, the phase shift of the reception signal periodically changes over a relatively long period. Thus, the addition result of the absolute values of the phase shift becomes relatively large at such a depth, and, at the depth where the fluctuation does not occur, the addition result of the absolute values of the phase shift is relatively small because a period where the phase shift of the reception signal is 0 (zero) is dominant in these depths. Thus, for example, the fluctuation detector 40 may add the absolute values of the phase shift over a plurality of times for each depth, and may judge that the fluctuation occurred at a certain depth when the addition result obtained for the depth exceeds a judgment threshold. Alternatively, an image portion (depth) where the fluctuation occurs may be judged based on an image analysis process for the time-space map.

The fluctuation detector 40 detects the depth where the fluctuation occurs, in each of the time-space map of the ultrasound beam T1 and the time-space map of the ultrasound beam T2. A depth where the fluctuation occurs in at least one of the time-space maps of the ultrasound beam T1 and the ultrasound beam T2 is communicated to the velocity evaluator 60.

Next, a specific example of shear wave measurement by the ultrasound diagnostic apparatus of FIG. 1 will be described. In the measurement of shear wave, the propagation velocity Vs of the shear wave is measured by the measurement sequence described above with reference to FIG. 2. The shear wave velocity calculator 50 calculates the propagation velocity Vs of the shear wave for each depth in the subject based on the time-space maps (refer to FIG. 3) of the ultrasound beam T1 and the ultrasound beam T2 of the tracking wave. With this process, a measurement value array including a plurality of propagation velocities Vs corresponding to a plurality of depths is obtained. Further, in the measurement of the shear wave, the measurement sequence described above with reference to FIG. 2 is executed for a plurality of times, a measurement set including a plurality of times of measurement sequences is executed, and a plurality of measurement value arrays corresponding to the measurement sequences of a plurality of times are obtained.

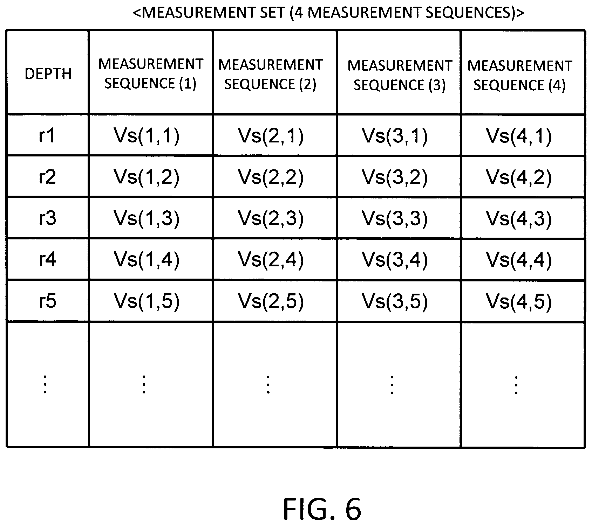

FIG. 6 is a diagram showing a specific example of a measurement result of a measurement set. FIG. 6 shows a measurement value array of the propagation velocities Vs obtained by a measurement sequence of 4 times. In the specific example of FIG. 6, for example, a measurement value array including a plurality of propagation velocities Vs(1, 1), Vs(1, 2), ... corresponding to a plurality of depths r1, r2, ... is obtained by a measurement sequence (1) of a first time, and a measurement value sequence including a plurality of propagation velocities Vs(2, 1), Vs(2, 2), ... corresponding to the plurality of depths r1, r2, ... is obtained by a measurement sequence (2) of a second time. Alternatively, a measurement set including measurement sequences of 5 times or more or 3 times or less may be executed.

After the measurement set including measurement sequences of a plurality of times is executed and a plurality of measurement values (a plurality of propagation velocities Vs) of the measurement set are calculated by the shear wave velocity calculator 50, the velocity evaluator 60 identifies, from among the plurality of measurement values, at least one measurement value which satisfies a discard condition. As the discard condition, for example, a condition based on the size of the measurement value (propagation velocity Vs), a condition based on a tissue state in the subject, or the like is desirable.

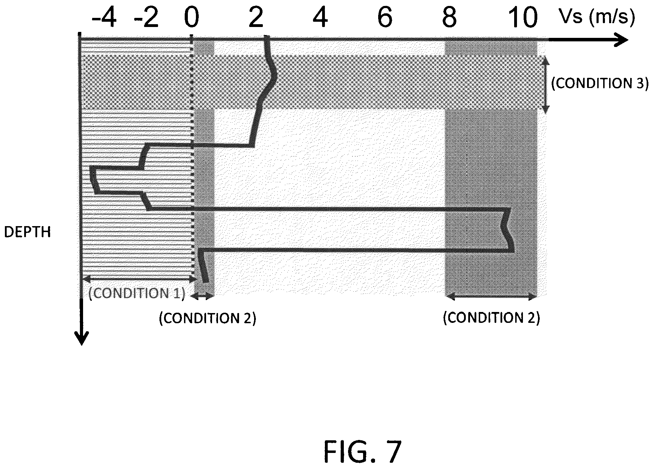

FIG. 7 is a diagram for explaining a specific example of the discard condition. FIG. 7 shows a velocity map for the propagation velocity Vs calculated by the shear wave velocity calculator 50. The velocity map of FIG. 7 corresponds to the propagation velocity Vs obtained by the measurement sequence of one time, a vertical axis represents the depth, and a horizontal axis represents the propagation velocity Vs. FIG. 7 shows conditions 1 to 3 as specific examples of the discard condition.

In condition 1, propagation velocities Vs having a negative sign (reverse direction) are set as discarding targets. For example, in the measurement of the shear wave described above with reference to FIG. 2, a relatively strong shear wave occurs at or near the position p to which the transmission beam P of the push wave is transmitted, and the shear wave propagates in the directions of two ultrasound beams T1 and T2 of the tracking wave. Therefore, if a direction in the specific example of FIG. 2 from the position p toward the position x1 is a positive (plus) direction, if the propagation velocity Vs of the shear wave is normal, the propagation velocity would take a positive (plus) value. However, when the shear wave cannot be normally detected due to disturbances in the shear wave or the like, it is possible that a propagation velocity Vs in the negative (minus) direction results from the calculation. Therefore, propagation velocities Vs having a negative sign (reverse direction) are determined to be a result with a low reliability, and are set as the discarding targets.

Further, in the specific example of FIG. 7, in condition 2, propagation velocities Vs outside of a threshold range are set as discarding targets. The values that can be taken by the propagation velocity Vs of the shear wave tend to fit within a certain range that can be determined clinically, for example, according to the tissue or the like to be diagnosed. Thus, a threshold range is set based on, for example, a large number of clinical results, and propagation velocities Vs outside of the threshold range are assumed to be a result of a low reliability, and are set as discarding targets. For example, the threshold range may be determined based on the type of the tissue to be diagnosed, age and sex of the subject, or the like, or the threshold range may be set adjustable by a user (measurer) such as a doctor or an inspector.

In condition 3, propagation velocities Vs at each depth where the fluctuation is detected are set as the discarding targets. As described above with reference to FIG. 4, in the measurement of the propagation velocity Vs of the shear wave, there may be cases where the displacement of the tissue is periodically fluctuated due to a motion of micro-blood vessels or bloodstream in a measurement region (region of interest), and the periodic fluctuation affects the measurement of the propagation velocity Vs of the shear wave. For example, in the region (depth) where the fluctuation occurs, it is difficult to identify the change of the phase shift due to passage of the shear wave, and to measure the propagation velocity Vs of the shear wave. Even if the propagation velocity Vs of the shear wave can be measured at the region (depth) where the fluctuation occurs, the reliability of the measurement result is questionable. Therefore, the propagation velocity Vs at each depth where the fluctuation is detected is set as a discarding target, being a result having a low reliability. As already described, the depth where the fluctuation occurs is detected by the fluctuation detector 40. The propagation velocity Vs to be set as the discarding target is identified by the velocity evaluator 60 which is a desirable specific example of a measurement value processor.

The velocity evaluator 60 sets, from among the propagation velocities Vs calculated by the shear wave velocity calculator 50 such as, for example, the plurality of propagation velocities Vs in the measurement set shown in FIG. 6, propagation velocities Vs satisfying the discard condition as discarding targets. For example, propagation velocities Vs corresponding to any one of conditions 1 to 3 described above with reference to FIG. 7 are set as discarding targets. The propagation velocities Vs which are set as the discarding targets may be, for example, deleted from the measurement set shown in FIG. 6, or a flag or the like showing that the velocity is a discarding target may be correlated, without deleting the value (data) of the propagation velocity Vs.

The velocity evaluator 60 discards the propagation velocities Vs satisfying the discard condition among the plurality of propagation velocities Vs in the measurement set, and calculates a VsN (valid Vs ratio) which is a ratio of a plurality of propagation velocities Vs which are not discarded and which remain; that is, a plurality of propagation velocities Vs which are assumed to be valid measurement values.

The velocity evaluator 60 calculates the VsN for each measurement sequence in the measurement set. For example, in the measurement set shown in FIG. 6, for the propagation velocities Vs of a plurality of depths in each measurement sequence of measurement sequences (1) to (4), VsN (valid Vs ratio) is calculated for each measurement sequence. When, for example, the VsN of each measurement sequence is less than or equal to a threshold, the measurement sequence is assumed to have a low reliability, and the propagation velocities Vs of all depths of the measurement sequence may be discarded. For example, in the specific example of FIG. 6, when the VsN of the measurement sequence (3) is less than or equal to 30 percent which is the threshold, all propagation velocities Vs(3, 1), Vs(3, 2), ... of the measurement sequence (3) are discarded.

Further, the velocity evaluator 60 calculates a statistical value related to the propagation velocity Vs based on the plurality of the propagation velocities Vs which are not discarded and which remain; that is, the plurality of propagation velocities assumed to be valid measurement values, among the plurality of propagation velocities Vs in the measurement set. As the statistical value, for example, an average, a central value, an IQR, a standard deviation, a VsN (valid Vs ratio), or the like related to the plurality of propagation velocities Vs assumed to be valid measurement values are desirably used, but alternatively, other statistical values may be calculated. The calculated statistical value is displayed on the display 72 by, for example, numerical values.

In addition, the velocity evaluator 60 may form a histogram related to the propagation velocity Vs based on the plurality of propagation velocities Vs which are not discarded and which remain; that is, the plurality of propagation velocities assumed to be valid measurement values, among the plurality of propagation velocities Vs in the measurement set.

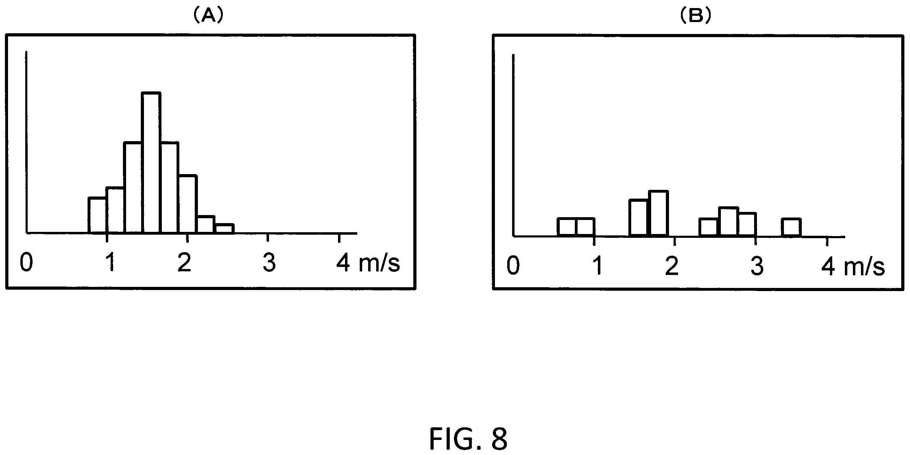

FIG. 8 is a diagram showing a specific example of the histogram related to the propagation velocity Vs. FIG. 8(A) shows a specific example of a histogram obtained in a measurement having a relatively large VsN (valid Vs ratio) and which is stable, and the distribution is unimodal. On the other hand, FIG. 8(B) shows a specific example of a histogram obtained in a measurement having a relatively small VsN and which is unstable, and the distribution is dispersive. In the histograms of FIGs. 8(A) and 8(B), a horizontal axis shows the propagation velocity Vs and a vertical axis shows an occurrence frequency. A maximum value for the vertical axis (occurrence frequency) is desirably determined by, for example, the following formula:

[Formula 1]

$$Y_{max} = ceil\left(\frac{N \cdot \Delta BIN}{std} \cdot \sigma\right)$$

where Ymax represents a maximum value of the occurrence frequency of the vertical axis; ceil(·) represents an operation to round up a fraction; N represents a number of values Vs before discarding; ΔBIN represents a bin width of the histogram; std represents an assumed standard deviation of Vs; and σ represents 1σ when a normal distribution is presumed.

Formula (1) is one of specific examples for standardizing the occurrence frequency on the vertical axis of the propagation velocity Vs. In Formula (1), it is assumed that a normal distribution is obtained when the propagation velocity Vs is ideally stably measured, and the maximum value (Ymax) of the occurrence frequency on the vertical axis is calculated based on the bin width (ΔBIN) of the histogram, and the assumed standard deviation (std) of the propagation velocity Vs.

For example, by standardizing the occurrence frequency on the vertical axis by Formula 1, the area of the histogram changes according to the number of valid propagation velocities Vs to be shown in the histogram (the plurality of propagation velocities Vs which are not discarded).

In the histograms shown in FIG. 8, the maximum values of the occurrence frequency on the vertical axis are determined based on Formula (1). The histogram of FIG. 8(A) is obtained by a stable measurement with a relatively large VsN (valid Vs ratio), and the number of valid propagation velocities Vs is relatively large. Thus, the area of the histogram is relatively large. On the other hand, the histogram of FIG. 8(B) is obtained by an unstable measurement having a relatively low VsN (valid Vs ratio). Thus, the number of valid propagation velocities Vs is relatively small, and the area of the histogram is consequently relatively small.

In this manner, by standardizing the maximum value of the occurrence frequency on the vertical axis based on the Formula (1), the number of valid propagation velocities Vs to be reflected in the histogram is reflected in the area of the histogram. Thus, it becomes possible to visually determine whether or not the measurement result is stable from the area of the histogram.

The histogram formed by the velocity evaluator 60, for example, the histogram shown in FIG. 8, is displayed on the display 72. The histogram may be displayed along with the B-mode image.

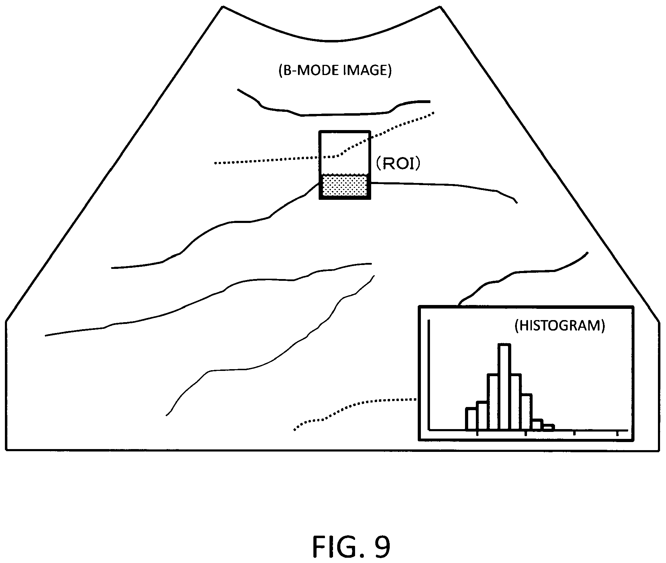

FIG. 9 is a diagram showing a specific example of a display image. FIG. 9 shows a specific example of a display image formed by the display processor 70 and displayed on the display 72. The display image of FIG. 9 is obtained based on the B-mode image (tomographic image) formed by the image former 20, and the histogram formed by the velocity evaluator 60.

In the B-mode image, a region of interest (ROI) may be displayed. For example, as shown in the specific example of FIG. 9, a quadrangular mark showing the region of interest (ROI) is displayed. The region of interest (ROI) is a region in which the measurement of the shear wave is executed; that is, the region where the time-space map (FIG. 3) is obtained.

Further, in the region of interest (ROI), a region corresponding to the fluctuation portion detected by the fluctuation detector 40 may be explicitly shown. For example, in the region of interest (ROI), the fluctuation portion is displayed in an emphasized manner by a display form such as a pattern, a brightness, a color, or the like. With such a configuration, it may become possible, for example, when the fluctuation portion is large (wide) in the region of interest (ROI), to allow the user to re-set a position of the region of interest (ROI).

In the specific example of FIG. 9, the histogram of the propagation velocity Vs (FIG. 8) is displayed over the B-mode image. The histogram may be displayed in a manner to not overlap the B-mode image, or, for example, the display and non-display may be switched according to an instruction from the user. Alternatively, the histogram alone may be displayed in a large size.

In addition, numerical values such as the statistical value related to the propagation velocity Vs calculated by the velocity evaluator 60 (such as the average, the central value, the IQR, the standard deviation, the VsN, or the like related to the valid propagation velocities Vs) may be displayed over or near the B-mode image.

An embodiment of the present disclosure has been described. The above-described embodiment, however, is merely exemplary in every aspect, and does not limit the scope of the present disclosure. The present disclosure includes various modifications within the scope of the invention as defined in the appended claims.

REFERENCE SIGNS LIST

10 PROBE; 12 TRANSMISSION UNIT; 14 RECEPTION UNIT; 20 IMAGE FORMER; 30 DISPLACEMENT MEASUREMENT UNIT; 40 FLUCTUATION DETECTOR; 50 SHEAR WAVE VELOCITY CALCULATOR; 60 VELOCITY EVALUATOR; 70 DISPLAY PROCESSOR; 72 DISPLAY; 80 CONTROL UNIT.

**Claims**

1. An ultrasound diagnostic apparatus comprising:

   an ultrasound probe (1) that is adapted to transmit an ultrasound push wave to generate a shear wave in a subject, and that is adapted to transmit an ultrasound tracking wave to the subject;
   a shear wave measurement unit (30) that is adapted to obtain a measurement value array including a measurement value at each depth of a plurality of depths within the subject by measuring the shear wave based on a reception signal obtained by transmitting the tracking wave; and
   a measurement value processor (70) that is adapted to identify a measurement value which satisfies a discard condition from among a plurality of the measurement value arrays obtained by executing the measurement of the shear wave a plurality of times,
   **characterised in that**
   the ultrasound diagnostic apparatus further comprises a detector (40) that is adapted to detect a fluctuation which is a periodic displacement of a tissue in the subject, based on the reception signal,
   the discard condition includes a condition to discard a measurement value of each depth at which the fluctuation is detected,
   the measurement value processor (70) is adapted to discard the measurement value of each depth in which the fluctuation is detected, from among the plurality of measurement values of the plurality of measurement value arrays,
   the discard condition includes a condition to discard a propagation velocity measurement value which is either negative or outside of a threshold range, and
   the measurement value processor (70) is adapted to discard the propagation velocity measurement value which is either negative or outside of the threshold range, from among the plurality of measurement values of the plurality of measurement value arrays.

2. The ultrasound diagnostic apparatus according to Claim 1, wherein the measurement value processor (70) is adapted to discard a measurement value which satisfies the discard condition, from among the plurality of measurement values of the plurality of measurement value arrays, and calculate a ratio of a plurality of measurement values which are not discarded and which remain.

3. The ultrasound diagnostic apparatus according to Claim 1 or 2, wherein the measurement value processor (70) is adapted to discard a measurement value which satisfies the discard condition, from among the plurality of measurement values of the plurality of measurement value arrays, and calculate a statistical value based on a plurality of measurement values which are not discarded and which remain.

4. The ultrasound diagnostic apparatus according to any one of Claims 1 to 3, wherein the measurement value processor (70) is adapted to discard a measurement value which satisfies the discard condition, from among the plurality of measurement values of the plurality of measurement value arrays, and form a histogram of a plurality of measurement values which are not discarded and which remain.

**Patentansprüche**

1. Ultraschalldiagnosegerät mit
   einer Ultraschallsonde (1), die dazu ausgelegt ist, eine Ultraschallkompressionswelle zu senden, um eine Scherwelle in einem Subjekt zu erzeugen, und die dazu ausgelegt ist, eine Ultraschallnachfolgewelle an das Objekt zu senden,
   einer Scherwellenmesseinheit (30), die dazu ausgelegt ist, einen Messwerte-Array zu erhalten, der einen Messwert in jeder Tiefe von mehreren Tiefen innerhalb des Subjekts enthält, indem die Scherwelle basierend auf einem durch Senden der Nachfolgewelle erhaltenen Empfangssignal gemessen wird, und
   einem Messwertprozessor (70), der dazu ausgelegt ist, einen Messwert zu identifizieren, der eine Ablehnungsbedingung von mehreren Messwerte-Arrays erfüllt, die durch mehrmaliges Durchführen der Messung der Scherwelle erhalten wurden,
   **dadurch gekennzeichnet, dass**
   das Ultraschalldiagnosegerät ferner einen Detektor (40) aufweist, der dazu ausgelegt ist, basierend auf dem Empfangssignal eine Schwankung zu erfassen, die eine periodische Verschiebung eines Gewebes in dem Subjekt ist,
   die Ablehnungsbedingung eine Bedingung zum Ablehnen eines Messwerts jeder Tiefe enthält, in der die Schwankung erfasst wurde,
   der Messwertprozessor (70) dazu ausgelegt ist, den Messwert in jeder Tiefe abzulehnen, in der die Schwankung unter den mehreren Messwerten der mehreren Messwerte-Arrays erfasst wurde,
   die Ablehnungsbedingung eine Bedingung enthält, um einen Ausbreitungsgeschwindigkeitsmesswert abzulehnen, der entweder negativ oder außerhalb eines Schwellenwertbereichs ist, und
   der Messwertprozessor (70) dazu ausgelegt ist, den Ausbreitungsgeschwindigkeitsmesswert unter den mehreren Messwerten der mehreren Messwerte-Arrays abzulehnen, der entweder negativ oder außerhalb des Schwellenwertbereichs ist.

2. Ultraschalldiagnosegerät nach Anspruch 1, wobei der Messwertprozessor (70) dazu ausgelegt ist, einen Messwert abzulehnen, der unter den mehreren Messwerten der mehreren Messwerte-Arrays die Ablehnungsbedingung erfüllt, und ein Verhältnis von mehreren Messwerten zu berechnen, die nicht abgelehnt wurden und die verbleiben.

3. Ultraschalldiagnosegerät nach Anspruch 1 oder 2, wobei der Messwertprozessor (70) dazu ausgelegt ist, einen Messwert abzulehnen, der unter den mehreren Messwerten der mehreren Messwerte-Arrays die Ablehnungsbedingung erfüllt, und einen statistischen Wert basierend auf mehreren Messwerten zu berechnen, die nicht abgelehnt wurden und die verbleiben.

4. Ultraschalldiagnosegerät nach einem der Ansprüche 1 bis 3, wobei der Messwertprozessor (70) dazu ausgelegt ist, einen Messwert abzulehnen, der unter den mehreren Messwerten der mehreren Messwerte-Arrays die Ablehnungsbedingungen erfüllt, und ein Histogramm von mehreren Messwerten zu bilden, die nicht abgelehnt wurden und die verbleiben.

**Revendications**

1. Appareil de diagnostic par ultrasons comprenant :

   une sonde à ultrasons (1) qui est adaptée à transmettre une onde ultrasonore de poussée pour générer une onde de cisaillement dans un sujet, et qui est adaptée à transmettre une onde ultrasonore de suivi jusqu'au sujet ;
   une unité (30) de mesure d'onde de cisaillement qui est adaptée à obtenir un ensemble de valeurs de mesure incluant une valeur de mesure à chaque profondeur d'une pluralité de profondeurs à l'intérieur du sujet en mesurant l'onde de cisaillement sur la base d'un signal de réception obtenu en transmettant l'onde de suivi ; et
   un processeur (70) de valeurs de mesure qui est adapté à identifier une valeur de mesure qui satisfait à une condition de rejet parmi une pluralité des ensembles de valeurs de mesure obtenus en exécutant la mesure de l'onde de cisaillement une pluralité de fois,
   **caractérisé en ce que**
   l'appareil de diagnostic par ultrasons comprend en outre un détecteur (40) qui est adapté à détecter une fluctuation qui est un déplacement périodique d'un tissu dans le sujet, sur la base du signal de réception,
   la condition de rejet inclut une condition pour rejeter une valeur de mesure de chaque profondeur à laquelle la fluctuation est détectée,
   le processeur (70) de valeurs de mesure est adapté à rejeter la valeur de mesure de chaque profondeur dans laquelle la fluctuation est détectée, parmi la pluralité de valeurs de mesure de la pluralité d'ensembles de valeurs de mesure,
   la condition de rejet inclut une condition pour rejeter une valeur de mesure de vitesse de propagation qui est soit négative, soit à l'extérieur d'une plage de seuil, et

le processeur (70) de valeurs de mesure est adapté à rejeter la valeur de mesure de vitesse de propagation qui est soit négative, soit à l'extérieur de la plage de seuil, parmi la pluralité de valeurs de mesure de la pluralité d'ensembles de valeurs de mesure.

2. Appareil de diagnostic par ultrasons selon la revendication 1, dans lequel le processeur (70) de valeurs de mesure est adapté à rejeter une valeur de mesure qui satisfait à la condition de rejet, parmi la pluralité de valeurs de mesure de la pluralité d'ensembles de valeurs de mesure, et calculer un rapport d'une pluralité de valeurs de mesure qui ne sont pas rejetées et qui subsistent.

3. Appareil de diagnostic par ultrasons selon la revendication 1 ou 2, dans lequel le processeur (70) de valeurs de mesure est adapté à rejeter une valeur de mesure qui satisfait à la condition de rejet, parmi la pluralité de valeurs de mesure de la pluralité d'ensembles de valeurs de mesure, et calculer une valeur statistique sur la base d'une pluralité de valeurs de mesure qui ne sont pas rejetées et qui subsistent.

4. Appareil de diagnostic par ultrasons selon l'une quelconque des revendications 1 à 3, dans lequel le processeur (70) de valeurs de mesure est adapté à rejeter une valeur de mesure qui satisfait à la condition de rejet, parmi la pluralité de valeurs de mesure de la pluralité d'ensembles de valeurs de mesure, et former un histogramme d'une pluralité de valeurs de mesure qui ne sont pas rejetées et qui subsistent.

FIG. 1

(A)

(B)

FIG. 2

EP 3 269 308 B1

<TIME-SPACE MAP>

FIG. 3

EP 3 269 308 B1

<TIME-SPACE MAP (WITH FLUCTUATION)>

DEPTH (mm)

TIME (ms)

FIG. 4

FIG. 5

EP 3 269 308 B1

<MEASUREMENT SET (4 MEASUREMENT SEQUENCES)>

| DEPTH | MEASUREMENT SEQUENCE (1) | MEASUREMENT SEQUENCE (2) | MEASUREMENT SEQUENCE (3) | MEASUREMENT SEQUENCE (4) |
|---|---|---|---|---|
| r1 | Vs(1,1) | Vs(2,1) | Vs(3,1) | Vs(4,1) |
| r2 | Vs(1,2) | Vs(2,2) | Vs(3,2) | Vs(4,2) |
| r3 | Vs(1,3) | Vs(2,3) | Vs(3,3) | Vs(4,3) |
| r4 | Vs(1,4) | Vs(2,4) | Vs(3,4) | Vs(4,4) |
| r5 | Vs(1,5) | Vs(2,5) | Vs(3,5) | Vs(4,5) |
| ⋮ | ⋮ | ⋮ | ⋮ | ⋮ |

FIG. 6

EP 3 269 308 B1

FIG. 7

(A)

(B)

FIG. 8

FIG. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8118744 B
- US 2013218011 A1
- US 2015005633 A1
- WO 2016060146 A1